# EUROPEAN PATENT APPLICATION

(11) **EP 4 151 226 A1**
(43) Date of publication of application: **22.03.2023**
(21) Application number: 21804054.1
(22) Date of filing: 13.05.2021
(51) Int. Cl.: A61K 36/758, A61K 31/7048, A61K 31/11, A61K 31/045, A61K 31/194, A61P 31/14, A23L 33/105

(54) **CORONAVIRUS THERAPEUTIC AGENT COMPRISING ZANTHOXYLUM PIPERITUM LEAF EXTRACT AS ACTIVE INGREDIENT**

(30) Priority: 13.05.2020 KR 20200057241; 12.05.2021 KR 20210061165
(71) Applicant: Mecox Curemed Co., Ltd., Seoul 06744 (KR); Choongang Ocean Co., Ltd., Seoul 06300 (KR)
(72) Inventor: CHUNG, Jae Yong, Yongin-si Gyeonggi-do 16930 (KR); LEE, Pil Goo, Yongin-si Gyeonggi-do 17004 (KR)
(74) Representative: WSL Patentanwälte Partnerschaft mbB
(86) International application number: PCT/KR2021/005978
(87) International publication number: WO 2021/230667

(57) **Abstract**

The present invention relates to a pharmaceutical composition for preventing, alleviating, or treating coronavirus infection, and a food composition for preventing or ameliorating coronavirus infection, comprising a *Zanthoxylum piperitum* leaf extract, a fraction thereof, or a compound isolated therefrom as an active ingredient. According to the present invention, the *Zanthoxylum piperitum* leaf extract, the fraction thereof, or quercetin-3-O-alpha-L-rhamnoside and/or kaempferol-3-O-alpha-L-rhamnoside, which are active compounds isolated from the *Zanthoxylum piperitum* leaf extract, exhibit(s) excellent antiviral activity against coronavirus, which is a virus that causes respiratory diseases, digestive diseases, liver disease, brain disease, etc. in mammals, in particular corona-19 virus, and thus can be useful in medicines and food for preventing, ameliorating, or treating diseases caused by coronavirus infection.

## Description

### [Technical Field]

The present disclosure relates to a pharmaceutical composition for preventing, alleviating, or treating coronavirus infection and a food composition for preventing or ameliorating coronavirus infection, each of which contains, as an active ingredient, a *Zanthoxylum piperitum* leaf extract, a fraction thereof, or a compound separated therefrom.

### [Background Art]

Coronavirus is an RNA virus that causes respiratory diseases, digestive diseases, liver diseases, brain diseases, etc. in mammals and birds (Gallagher T.M. et al., Virology, 279(2): 371-374, 2001). In particular, among viruses belonging to *Coronaviridae,* porcine transmissible gastroenteritis virus (TGEV) and porcine epidemic diarrhea virus (PEDV), which induce highly contagious viral diseases, are viruses that invade the gastrointestinal tract and digestive system, causing dehydration and high fever due to vomiting and diarrhea and incurring considerable economic loss due to a high fatality rate (Duarte M. et al., J. Gen. Virol., 75(Pt 5):1195-1200, 1994). Although these viruses have a very high fatality rate, no therapeutic agents have been developed like diseases caused by other virus infections. Therefore, vaccine development studies for preventing virus infection are in progress, but effectiveness thereof is still low (Alonso S. et al., J. Gen. Virol., 83(Pt 3):567-579, 2002).

Coronavirus-based COVID-19 (coronavirus disease 2019) or Corona-19 is a respiratory infection caused by a new type of coronavirus (SARS-CoV-2; RNA virus belonging to *Coronaviridae*) that first emerged in Wuhan, China in December 2019 and then has spread throughout China and around the world. It was initially known only as a respiratory infectious disease of unknown cause, but the pathogen thereof was identified as the World Health Organization (WHO) on January 9, 2020 announced that the cause of the pneumonia was a new type of coronavirus (SARS-CoV-2, named February 11 by the International Committee on Taxonomy of Viruses).

The pathogen of COVID-19 is 'SARS-CoV-2'. The International Committee on Taxonomy of Viruses (ICTV) published a paper on February 11, 2020, proposing the name SARS-CoV-2 for the pathogen of Corona-19. The committee said it emphasized that this virus is similar to the SARS (severe acute respiratory syndrome) virus of the outbreak of 2003.

The Korea Disease Control and Prevention Agency (KCDC) obtained and analyzed the gene sequence of the virus disclosed by China through academia, and confirmed that it had the highest homology (89.1%) with bat-derived coronavirus. Homology with 4 human coronaviruses was low at 39%-43%, and homology with MERS was confirmed at 50% and with SARS at 77.5%.

Corona-19 is transmitted when droplets (saliva) of an infected person infiltrate the respiratory system or mucous membrane of the eyes, nose, and mouth. For droplet infection, when an infected person coughs or sneezes, viruses and bacteria are mixed with small droplets such as saliva to infect others. Here, the typical travel distance thereof is known to be 2 m. In the case of eyes, if a patient's saliva or the like enters the eyes directly or the eyes are rubbed with hands contaminated with the virus, Corona-19 may be transmitted through the eyes. On February 19, Chinese authorities first recognized the possibility of transmission of Corona-19 by solid or liquid particles floating in the air, namely aerosols.

Upon being infected with Corona-19, after an incubation period of about 2 to 14 days (estimated), major symptoms, including fever (37.5°C), respiratory symptoms such as cough or shortness of breath, and pneumonia, appear. Moreover, muscle pain, fatigue, and diarrhea may occur, but there are rare cases of asymptomatic infection.

Currently, vaccines or therapeutic agents for Corona-19 have not yet been developed, and if confirmed as a Corona-19 patient, symptomatic treatment (symptomatic therapy) such as administration of antiviral drugs or antibiotics to prevent secondary infection is performed depending on major symptoms such as cough, sore throat, pneumonia, and the like.

Meanwhile, *Zanthoxylum piperitum* is a deciduous shrub of the Rutaceae family and is mainly distributed in Korea, Japan, and China. The fruit bark (pericarp) of *Zanthoxylum piperitum* is usually used as a spice and medicine, and seeds, young leaves, trees, and stems thereof are also used for various purposes. *Zanthoxylum piperitum* fruit is used as edible oil because it contains a lot of oil. In oriental medicine, it is used as an aromatic stomachic, intestinal metabolism stimulator, antidote, anthelmintic, and analgesic, and is referred to as *Zanthoxyli fructus* (not *Zanthoxylum schinifolium*) or fruit of *Zanthoxylum schinifolium.* Moreover, according to oriental medicine, it is said that a decoction of leaves of *Zanthoxylum piperitum* is good for arteriosclerosis, tooth decay, and periodontitis, but the specific effect thereof has not yet been scientifically identified.

Recently, *Zanthoxylum piperitum* is used as the world's best natural spice surpassing pepper and mustard, and conventional research has been conducted mainly targeting the fruit bark, root, and xylem of *Zanthoxylum piperitum.* It is known that the extract thereof exhibits antibacterial activity, insecticidal activity, anticancer activity, anti-inflammatory activity, effects on arteriosclerosis, hyperlipidemia, diabetes, and osteoporosis, and skin-whitening efficacy. Specifically, conventional techniques using *Zanthoxylum piperitum* include a medicinal liquor using herbs (the pericarp of *Zanthoxylum piperitum* and *Zanthoxylum schinifolium*) as a main material and a method of manufacturing the same as disclosed in Korean Patent Application Publication No. 10-1997-0002913, an antibiotic material separated from *Zanthoxylum piperitum* and a method of separating the same as disclosed in Korean Patent No. 10-0314545, a pharmaceutical composition and a functional health food for treating tuberculosis containing a crude *Zanthoxyli fructus* extract as an active ingredient as disclosed in Korean Patent No. 10-0656969, a composition for the prevention and treatment of cardiovascular diseases containing *Zanthoxylum piperitum* extracts or compounds isolated therefrom as disclosed in Korean Patent No. 10-0883992, a composition having anti-allergic activity containing a *Zanthoxylum piperitum* fruit extract or a glycoprotein isolated therefrom as disclosed in Korean Patent No. 10-1182824, and the like.

However, in any literature published to date, there is no disclosure that *Zanthoxylum piperitum* leaf extract is effective for the prevention, amelioration, or treatment of coronavirus infection, particularly COVID-19. Furthermore, the *Zanthoxylum piperitum* leaf extract contains quercetin-3-O-alpha-L-rhamnoside and kaempferol-3-O-alpha-L-rhamnoside as indicators and active compounds, and is thus regarded as different from conventional *Zanthoxylum piperitum* fruit bark, root, and xylem extracts. In addition, there is no disclosure in any literature that quercetin-3-O-alpha-L-rhamnoside or kaempferol-3-O-alpha-L-rhamnoside has anti-coronavirus efficacy, particularly therapeutic efficacy against COVID-19.

### [Citation List]

### [Patent Literature]

Korean Patent Application Publication No. 10-1997-0002913
Korean Patent No. 10-0314545
Korean Patent No. 10-0656969
Korean Patent No. 10-0883992
Korean Patent No. 10-1182824

### [Non-Patent Literature]

Gallagher T.M. et al., Virology, 279(2): 371-374, 2001
Duarte M. et al., J. Gen. Virol., 75(Pt 5):1195-1200, 1994
Alonso S. et al., J. Gen. Virol., 83(Pt 3):567-579, 2002

### [Disclosure]

### [Technical Problem]

An object of the present invention is to provide a pharmaceutical composition for preventing, alleviating, or treating coronavirus infection containing a *Zanthoxylum piperitum* leaf extract, a fraction thereof, or a compound separated therefrom as an active ingredient.

Another object of the present invention is to provide a food composition for preventing or ameliorating coronavirus infection containing a *Zanthoxylum piperitum* leaf extract, a fraction thereof, or a compound separated therefrom as an active ingredient.

The objects of the present invention are not limited to the foregoing. The objects of the present invention will be able to be clearly understood through the following description and to be realized by the means described in the claims and combinations thereof.

### [Technical Solution]

In order to accomplish the above objects, the following solutions are provided.

An embodiment of the present invention provides a pharmaceutical composition for preventing, alleviating, or treating coronavirus infection containing a *Zanthoxylum piperitum* leaf extract, a fraction thereof, or a compound separated therefrom as an active ingredient.

In an embodiment of the present invention, the extract may be extracted with water, a C₁-C₄ lower alcohol, or a mixed solvent thereof.

In an embodiment of the present invention, the lower alcohol may be ethanol.

In an embodiment of the present invention, the extract may be extracted with ethanol or a 60% to 99% ethanol aqueous solution.

In an embodiment of the present invention, the extract may be extracted with a 90% to 99% ethanol aqueous solution.

In an embodiment of the present invention, the fraction may be an ethyl acetate fraction obtained by suspending the *Zanthoxylum piperitum* leaf extract in distilled water and then performing sequential fractionation with n-hexane, dichloromethane, ethyl acetate, and *n*-butanol.

In an embodiment of the present invention, the *Zanthoxylum piperitum* leaf extract or the ethyl acetate fraction may include, as an active ingredient, at least one compound selected from the group consisting of quercetin-3-O-alpha-L-rhamnoside represented by [Chemical Formula 1] below, kaempferol-3-O-alpha-L-rhamnoside represented by [Chemical Formula 2] below, 4'-hydroxyacetophenone represented by [Chemical Formula 3] below, 2,4-di-tert-butylphenol represented by [Chemical Formula 4] below, and 1,2-benzenedicarboxylic acid represented by [Chemical Formula 5] below.

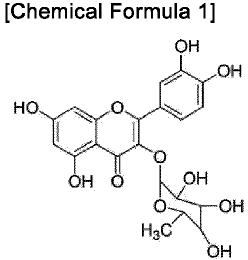

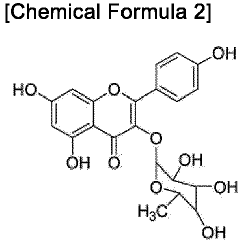

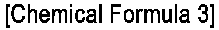

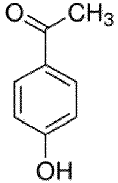

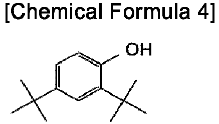

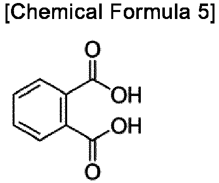

In an embodiment of the present invention, the coronavirus may be at least one selected from the group consisting of porcine transmissible gastroenteritis virus (TGEV), porcine epidemic diarrhea virus (PEDV), canine coronavirus, bovine coronavirus, SARS coronavirus (SARS-CoV), and Corona-19 virus (SARS-CoV-2).

In an embodiment of the present invention, the pharmaceutical composition may include a pharmaceutically acceptable carrier, excipient, or diluent.

In an embodiment of the present invention, the pharmaceutical composition may be administered orally in any form selected from the group consisting of a powder, a granule, a tablet, a capsule, and a liquid form.

Another embodiment of the present invention provides a food composition for preventing or ameliorating coronavirus infection containing a *Zanthoxylum piperitum* leaf extract, a fraction thereof, or a compound separated therefrom as an active ingredient.

### [Advantageous effects]

According to the present invention, a *Zanthoxylum piperitum* leaf extract, a fraction thereof, or an active compound separated therefrom such as quercetin-3-O-alpha-L-rhamnoside and/or kaempferol-3-O-alpha-L-rhamnoside exhibits excellent antiviral activity against coronavirus, particularly Corona-19 virus, which is a virus that causes respiratory diseases, digestive diseases, liver diseases, brain diseases, etc. in mammals, and can thus be efficiently used in medicaments and foods for the prevention, amelioration, or treatment of diseases caused by coronavirus infection.

### [Description of Drawings]

FIG. 1 shows a process of obtaining solvent fractions and active compounds from a *Zanthoxylum piperitum* leaf extract according to an embodiment of the present invention;

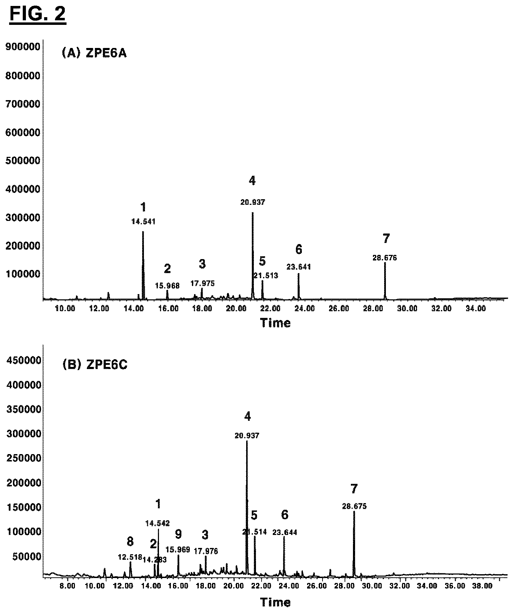

FIG. 2 shows GC-MS spectra of (A) subfraction ZPE6A and (B) subtraction ZPE6C obtained according to an embodiment of the present invention;
FIG. 3 shows results of evaluation of cytotoxicity of a *Zanthoxylum piperitum* 95% ethanol aqueous solution extract in MDCK and BEAS-2B cells in Example 4;
FIG. 4 shows results of primary plaque assay in Example 5;
FIG. 5 shows results of secondary plaque assay in Example 5;
FIG. 6 shows results of plaque assay of the *Zanthoxylum piperitum* extract at concentrations of 5 µg/mL or less in Example 5;
FIG. 7 shows results of plaque assay of the *Zanthoxylum piperitum* extract at concentrations of 5 µg/mL or less in Example 5;
FIG. 8 shows results of evaluation of inhibitory efficacy of the *Zanthoxylum piperitum* 95% EtOH extract on viral protein expression in MDCK cells in Example 6;
FIG. 9 shows results of evaluation of inhibitory efficacy of the *Zanthoxylum piperitum* 95% EtOH extract on IκBα degradation by LPS in Raw264.7 cells in Example 7;
FIG. 10 shows results of evaluation of inhibitory efficacy of the *Zanthoxylum piperitum* 95% EtOH extract on TNF-α mRNA expression by virus in Raw264.7 cells in Example 8;
FIG. 11 shows results of evaluation of inhibitory efficacy of the *Zanthoxylum piperitum* 95% EtOH extract on IL-6 mRNA expression by virus in Raw264.7 cells in Example 8; and
FIG. 12 shows results of evaluation of inhibitory efficacy of the *Zanthoxylum piperitum* 95% EtOH extract on IL-1β mRNA expression by virus in Raw264.7 cells in Example 8.

### [Best Mode]

Unless otherwise specified, all numbers, values, and/or representations that express components, reaction conditions, the amounts of components used herein are to be taken as approximations including various uncertainties affecting measurement that inherently occur in obtaining these values, among others, and thus should be understood to be modified by the term "about" in all cases. Furthermore, when a numerical range is disclosed in this specification, the range is continuous, and includes all values from the minimum value of said range to the maximum value thereof, unless otherwise indicated. Moreover, when such a range pertains to integer values, all integers including the minimum value to the maximum value are included, unless otherwise indicated.

In the present specification, when a range is described for a variable, it will be understood that the variable includes all values including the end points described within the stated range. For example, the range of "5 to 10" will be understood to include any subranges, such as 6 to 10, 7 to 10, 6 to 9, 7 to 9, and the like, as well as individual values of 5, 6, 7, 8, 9 and 10, and will also be understood to include any value between valid integers within the stated range, such as 5.5, 6.5, 7.5, 5.5 to 8.5, 6.5 to 9, and the like. Also, for example, the range of "10% to 30%" will be understood to include subranges, such as 10% to 15%, 12% to 18%, 20% to 30%, etc., as well as all integers including values of 10%, 11%, 12%, 13% and the like up to 30%, and will also be understood to include any value between valid integers within the stated range, such as 10.5%, 15.5%, 25.5%, and the like.

Moreover, unless otherwise defined, terms and abbreviations used herein may be interpreted as having meanings commonly understood by those skilled in the art to which the present invention belongs.

The present inventors have made great efforts to search for natural materials having few side effects while being effective at preventing, ameliorating, or treating diseases caused by coronavirus infection, and ascertained that a *Zanthoxylum piperitum* leaf extract, a fraction thereof, or an active compound separated therefrom such as quercetin-3-O-alpha-L-rhamnoside and/or kaempferol-3-O-alpha-L-rhamnoside may exhibit excellent antiviral activity against coronavirus, particularly Corona-19 virus, which is a virus that causes respiratory diseases, digestive diseases, liver diseases, brain diseases, etc. in mammals, thus culminating in the present invention.

Hereinafter, a detailed description will be given of the present invention.

The present invention pertains to a pharmaceutical composition for preventing, alleviating, or treating coronavirus infection, particularly COVID-19 and a food composition for preventing or ameliorating coronavirus infection, each of which contains a *Zanthoxylum piperitum* leaf extract, a fraction thereof, or an active compound separated therefrom such as quercetin-3-O-alpha-L-rhamnoside and/or kaempferol-3-O-alpha-L-rhamnoside, as an active ingredient.

An aspect of the present invention pertains to a pharmaceutical composition for preventing, alleviating, or treating coronavirus infection containing a *Zanthoxylum piperitum* leaf extract, a fraction thereof, or a compound separated therefrom as an active ingredient.

Another aspect of the present invention pertains to a method of preventing, alleviating, or treating coronavirus infection in a subject including administering an effective amount of a *Zanthoxylum piperitum* leaf extract, a fraction thereof, or a compound separated therefrom to a subject in need thereof.

Still another aspect of the present invention pertains to the use of a *Zanthoxylum piperitum* leaf extract, a fraction thereof, or a compound separated therefrom for the manufacture of a medicament for the prevention, alleviation, or treatment of coronavirus infection.

Yet another aspect of the present invention pertains to a *Zanthoxylum piperitum* leaf extract, a fraction thereof, or a compound separated therefrom for use in the prevention, alleviation, or treatment of coronavirus infection.

The present invention is characterized by a series of methods for the separation of an extract, a fraction, and an active compound from *Zanthoxylum piperitum,* specifically including:
1) extracting *Zanthoxylum piperitum* leaves with an extraction solvent;
2) filtering the extract of step 1);
3) preparing a *Zanthoxylum piperitum* leaf extract by concentrating the filtered extract of step 2) under reduced pressure and then performing drying;
4) preparing a *Zanthoxylum piperitum* leaf fraction by additionally extracting the *Zanthoxylum piperitum* leaf extract of step 3) with an organic solvent; and
5) obtaining quercetin-3-O-alpha-L-rhamnoside represented by [Chemical Formula 1] below or kaempferol-3-O-alpha-L-rhamnoside represented by [Chemical Formula 2] below by performing silica gel column chromatography and medium pressure liquid chromatography (MPLC) on the fraction obtained in step 4), particularly an ethyl acetate fraction.

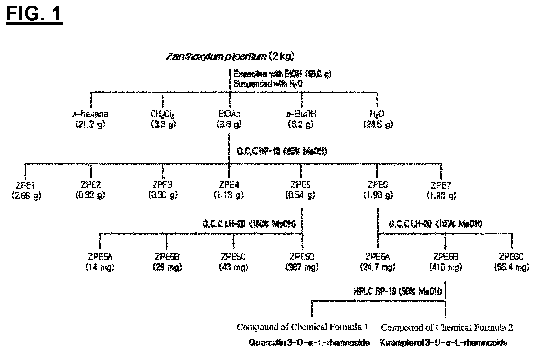

With reference to the process shown in FIG. 1, the method of separating an extract, solvent fractions, or active compounds from *Zanthoxylum piperitum* leaves is specified below.

In the method of the present invention, the *Zanthoxylum piperitum* leaves of step 1) may be used without limitation, so long as they are cultivated ones or commercially available ones. Also, although not limited thereto, the whole plant of *Zanthoxylum piperitum* including stems, branches, and roots may be extracted, in addition to the *Zanthoxylum piperitum* leaves in step 1).

In the method of the present invention, the extraction solvent in step 1) is preferably water, an alcohol, or a mixture thereof. The alcohol is preferably a C₁-C₄ lower alcohol, and the lower alcohol is preferably ethanol. The ethanol is preferably a 60% to 99% ethanol aqueous solution, more preferably a 90% to 99% ethanol aqueous solution, but is not limited thereto. The extraction method is preferably shaking extraction, Soxhlet extraction, or reflux extraction, but is not limited thereto. The extraction solvent is used in an amount corresponding to 1 to 30 times, 1 to 20 times, or 1 to 10 times the amount of dried *Zanthoxylum piperitum* leaves. The extraction temperature is preferably 20°C to 100°C, more preferably 20°C to 60°C, most preferably room temperature, but is not limited thereto. In addition, the extraction time is preferably 1 to 10 days, but is not limited thereto. In addition, extraction may be performed at least once, but the yield of the active ingredient may be remarkably decreased with an increase in the number of extraction processes, so it may not be economical to repeat extraction more than 5 times. Accordingly, extraction is preferably performed 1 to 5 times, more preferably 2 to 5 times, but the present invention is not limited thereto.

The extraction method may include a method commonly known in the art, for example, a method using an extraction device such as supercritical extraction, subcritical extraction, high-temperature extraction, high-pressure extraction, or ultrasonic extraction, a method using an adsorption resin such as XAD or HP-20, or the like.

In the method of the present invention, the concentration under reduced pressure in step 3) is preferably performed using a vacuum concentrator or a vacuum rotary evaporator, but the present invention is not limited thereto. In addition, the drying process is preferably performed through drying under reduced pressure, vacuum drying, boiling drying, spray drying, or freeze drying, but is not limited thereto.

In the method of the present invention, the organic solvent in step 4) is preferably n-hexane, dichloromethane (CH₂Cl₂), ethyl acetate (EtOAc), or butanol (BuOH), but is not limited thereto. The fraction is preferably any one selected from among an n-hexane fraction, a dichloromethane fraction, an ethyl acetate fraction, an n-butanol fraction, and a water fraction obtained by suspending the *Zanthoxylum piperitum* leaf extract in water and then performing sequential systematic fractionation with n-hexane, dichloromethane (CH₂Cl₂), ethyl acetate (EtOAc), n-butanol (n-BuOH), and water (H₂O), and is more preferably an ethyl acetate fraction, but is not limited thereto. The fraction may be obtained by subjecting the *Zanthoxylum piperitum* leaf extract to fractionation 1 to 5 times, preferably 3 times, and it is preferable to conduct a concentration process under reduced pressure after fractionation, but the present invention is not limited thereto.

In the method of the present invention, step 5) is separating and obtaining an active compound by performing silica gel column chromatography and medium pressure liquid chromatography (MPLC) on the fraction obtained in step 4), particularly the ethyl acetate fraction.

Specifically, the ethyl acetate fraction obtained in step 4) is placed in a glass column (5x50 cm) packed with silica beads, and silica gel column chromatography is performed. Here, a mixed solution of ethyl acetate and methanol is used as an eluent, and elution is performed while changing the volume ratio of ethyl acetate/methanol from 90/10 to 60/40. Based on the results of silica gel column chromatography, seven fractions were obtained, and the fractions thus obtained were named ZPE1, ZPE2, ZPE3, ZPE4, ZPE5, ZPE6, and ZPE7. Among the seven fractions obtained through silica gel column chromatography, ZPE6 showed the best activity, and thus, fraction ZPE6 was selected and medium pressure liquid chromatography (MPLC) was then performed.

Fraction ZPE6 that was selected was placed in a Sephadex LH-20 column and subjected to medium pressure liquid chromatography (MPLC). Here, elution is carried out using methanol alone as the eluent. Based on the results of medium pressure liquid chromatography (MPLC), three subfractions were obtained, and the subfractions thus obtained were named ZPE6A, ZPE6B, and ZPE6C. Among the three subfractions obtained through medium pressure liquid chromatography (MPLC), ZPE6B showed the best activity, and thus, subtraction ZPE6B was selected and active compounds were separated therefrom.

In order to separate the active compounds from subfraction ZPE6B, high-performance liquid chromatography (HPLC) using a reverse phase column packed with octadecyl-functionalized silica is performed. Here, a mixed solution of methanol and water is used as the eluent, and preferably, a 50% methanol aqueous solution containing methanol and water in equal amounts is used.

The active compounds separated through HPLC were analyzed through nuclear magnetic resonance spectroscopy, and identified to be quercetin-3-O-alpha-L-rhamnoside (quercitrin) represented by [Chemical Formula 1] below and kaempferol-3-O-alpha-L-rhamnoside (afzelin) represented by [Chemical Formula 2] below.

The chemical structures of active ingredient 1 and active ingredient 2 separated above were identified through nuclear magnetic resonance spectroscopy, respectively.

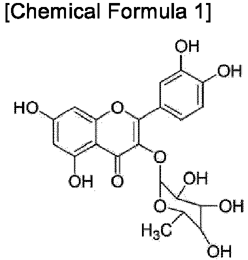

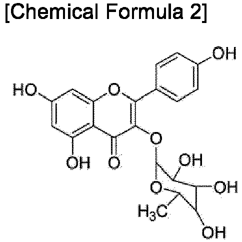

Also, although not limited thereto, the ethyl acetate (EtOAc) fraction may include, in addition to quercetin-3-O-alpha-L-rhamnoside represented by [Chemical Formula 1] and kaempferol-3-O-alpha-L-rhamnoside represented by [Chemical Formula 2] as an active ingredient, 4'-hydroxyacetophenone represented by [Chemical Formula 3] below, 2,4-di-tert-butylphenol represented by [Chemical Formula 4], and 1,2-benzenedicarboxylic acid represented by [Chemical Formula 5] below.

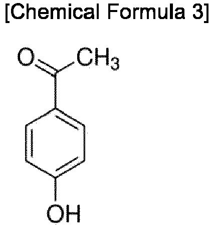

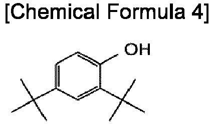

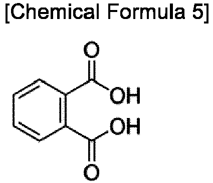

The *Zanthoxylum piperitum* leaf extract obtained through the separation method, the solvent fraction obtained by extracting the extract with the organic solvent, and quercetin-3-O-alpha-L-rhamnoside (Chemical Formula 1) and kaempferol-3-O-alpha-L-rhamnoside (Chemical Formula 2) separated therefrom were confirmed to show significant results against various coronaviruses, especially Corona-19 virus. Therefore, the *Zanthoxylum piperitum* leaf extract, the fraction thereof, and/or the active compound separated therefrom, such as the compound represented by [Chemical Formula 1] or [Chemical Formula 2] may be contained as an active ingredient in a pharmaceutical composition or a food composition for the prevention, amelioration, or treatment of a disease caused by coronavirus infection, particularly COVID-19.

In the present invention, the coronavirus may be at least one selected from the group consisting of porcine transmissible gastroenteritis virus (TGEV), porcine epidemic diarrhea virus (PEDV), canine coronavirus, bovine coronavirus, SARS coronavirus (SARS-CoV), and Corona-19 virus (SARS-CoV-2), but is not limited thereto.

In the present invention, the coronavirus is preferably Corona-19 virus (SARS-CoV-2).

The pharmaceutical composition according to the present invention contains the *Zanthoxylum piperitum* leaf extract, the fraction thereof, and/or the active compound separated therefrom as an active ingredient. Here, the active ingredient may be included in an amount of 0.01 to 80 wt% based on the total weight of the composition, but the present invention is not limited thereto.

The pharmaceutical composition of the present invention may further include a pharmaceutically acceptable carrier, excipient, and diluent. As used herein, the term "pharmaceutically acceptable" refers to exhibiting non-toxic properties in cells or humans exposed to the composition. The carrier may be used without limitation, so long as it is known in the art, examples thereof including buffers, preservatives, pain relievers, solubilizers, isotonic agents, stabilizers, bases, excipients, lubricants, and the like.

Moreover, the pharmaceutical composition of the present invention may be formulated in oral dosage forms such as powders, granules, tablets, capsules, suspensions, emulsions, syrups, and aerosols, or in forms such as external preparations, suppositories, and sterile injection solutions, according to typical methods. Examples of the carriers, excipients, and diluents that may be contained in the composition of the present invention may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. For formulation, diluents or excipients that are commonly used, such as fillers, extenders, binders, wetting agents, disintegrants, surfactants, and the like, may be used.

Examples of solid formulations for oral administration include tablets, pills, powders, granules, capsules, and the like, and such solid formulations are prepared by mixing the *Zanthoxylum piperitum* leaf extract, the fraction thereof, and/or the active compound separated therefrom with at least one excipient, for example, starch, calcium carbonate, sucrose, lactose, gelatin, or the like. In addition to simple excipients, lubricants such as magnesium stearate and talc are also used. Examples of liquid formulations for oral administration may include suspensions, oral liquids, emulsions, syrups, etc., and such liquid formulations may include various excipients, such as wetting agents, sweeteners, flavoring agents, preservatives, etc., in addition to simple diluents that are commonly used, such as water and liquid paraffin. Formulations for parenteral administration include sterile aqueous solutions, non-aqueous solvents, suspension agents, emulsions, freeze-dried preparations, and suppositories. Non-aqueous solvents and suspension agents include propylene glycol, polyethylene glycol, vegetable oils such as olive oil, and injectable esters such as ethyl oleate. As a base of the suppository, Witepsol, Macrogol, Tween 61, cacao butter, laurin butter, glycerogelatin, or the like may be used.

Also, the pharmaceutical composition of the present invention is administered in a pharmaceutically effective amount. The term "administration" of the present invention means introduction of a predetermined material to a subject through an appropriate method, and the composition may be administered through any general route, so long as it is able to reach a target tissue. Intraperitoneal administration, intravenous administration, intramuscular administration, subcutaneous administration, intradermal administration, oral administration, topical administration, intranasal administration, intrapulmonary administration, or intrarectal administration may be carried out, but the present invention is not limited thereto.

The term "subject" refers to all animals including humans, rats, mice, livestock, and the like. Preferably, the subject is a mammal including a human.

The term "pharmaceutically effective amount" refers to an amount sufficient to treat a disease at a reasonable benefit/risk ratio applicable to medical treatment and not to cause side effects, and the effective dosage level may be readily determined by those skilled in the art, depending on various factors including the patient's gender, age, body weight, and health status, the type of disease, the severity of disease, the drug activity, the sensitivity to drug, the method of administration, the time of administration, the route of administration, the rate of excretion, the treatment period, and drugs used in combination or concomitantly, and other factors well known in medicine. However, in order to realize a desired effect, for adults, the composition of the present invention may be used in an amount of 0.0001 to 1000 mg/kg, preferably 10 to 300 mg/kg (body weight) as a single dose. Here, the recommended dosage may be administered once a day or several times by dividing the same. The above dosage does not limit the scope of the present invention in any way.

A further aspect of the present invention pertains to a food composition for preventing or ameliorating coronavirus infection containing a *Zanthoxylum piperitum* leaf extract, a fraction thereof, or a compound separated therefrom as an active ingredient.

Here, the *Zanthoxylum piperitum* leaf extract, the fraction thereof, or the compound separated therefrom, and the effect of the *Zanthoxylum piperitum* leaf extract, the fraction thereof, or the compound separated therefrom on preventing, ameliorating, or treating coronavirus infection are as described above. When the composition of the present invention is used as a food additive, the *Zanthoxylum piperitum* leaf extract, the fraction thereof, or the compound separated therefrom may be added as is or used along with other foods or food ingredients, and may be appropriately used according to a typical method. The amount of the active ingredient that is mixed may be appropriately determined according to the intended use (preventive, health, or therapeutic purposes), and a culinarily acceptable food supplement additive may be further included. Since the composition of the present invention contains an extract derived from a natural material and a fraction thereof as an active ingredient, there is no problem in safety, so no great limitation is imposed on the mixed amount thereof.

The food composition of the present invention may include all foods in the ordinary sense, and may be used interchangeably with terms known in the art, such as functional food, functional health food, etc.

As used herein, the term "functional food" refers to a food manufactured and processed using raw materials or ingredients having useful functions in the human body in accordance with the Korean Health Functional Foods Act No. 6727, and "functional" means ingestion for the purpose of regulating nutrients or obtaining beneficial effects on health use such as physiological action for the structure and function of the human body.

In addition, the term "functional health food" as used herein refers to a food manufactured and processed by extracting, concentrating, refining, mixing, etc., a specific ingredient serving as a raw material or a specific ingredient contained in a food material for the purpose of health supplementation, and also refers to a food designed and processed to sufficiently exert biological control functions such as biological defense, regulation of biological rhythm, prevention of and recovery from disease, etc., by the above ingredient, and the composition for health food is responsible for functions related to disease prevention and disease recovery.

There is no limitation on the type of food in which the composition of the present invention may be used. In addition, the composition of the present invention containing the *Zanthoxylum piperitum* leaf extract, the fraction thereof, and/or the active compound separated therefrom as an active ingredient may be mixed with known additives and other suitable auxiliary ingredients that may be contained in food according to the choice of those skilled in the art. Examples of the food that may be added include meat, sausages, bread, chocolate, candy, snacks, confectioneries, pizza, ramen, other noodles, gum, dairy products including ice cream, various soups, beverages, tea, drinks, alcoholic beverages, and vitamin complexes, and the food may be prepared by adding the extract according to the present invention and the fraction thereof as a main ingredient to squeezed liquid, tea, jelly, juice, and the like.

In addition, examples of foods that may be applied to the present invention include special nutritional foods (e.g. infant formula, infant/toddler food, etc.), processed meat products, fish meat products, tofu, jelly, noodles (e.g. ramen, noodles, etc.), health supplements, seasoned foods (e.g. soy sauce, fermented soybean paste, red pepper paste, mixed paste, etc.), sauces, confectioneries (e.g. snacks), dairy products (e.g. fermented milk, cheese, etc.), other processed foods, kimchi, pickled foods (various types of kimchi, pickles, etc.), beverages (e.g. fruit beverages, vegetable beverages, soy milk, fermented beverages, etc.), and natural seasonings (e.g. ramen soup, etc.).

When the functional health food composition of the present invention is used in the form of a beverage, it may contain various sweeteners, flavoring agents, or natural carbohydrates as additional ingredients, as in typical beverages. In addition thereto, the functional health food composition of the present invention may contain various nutrients, vitamins, electrolytes, flavors, colorants, pectic acid and salts thereof, alginic acid and salts thereof, organic acids, protective colloidal thickeners, pH adjusters, stabilizers, preservatives, glycerin, alcohols, carbonation agents used in carbonated beverages, and the like. Moreover, it may contain pulp for the production of natural fruit juice, fruit juice beverage, and vegetable beverage.

Since the *Zanthoxylum piperitum* leaf extract according to the present invention, the fraction thereof, or the compound separated therefrom is obtained using a natural medicinal plant as a raw material, even when used for a pharmaceutical composition or a food composition, side effects may be less than when general synthetic compounds are used, so it may be safely contained and efficiently used.

### [Mode for Invention]

A better understanding of the present invention may be obtained through the following examples. These examples are merely set forth to illustrate the present invention, and are not to be construed as limiting the scope of the present invention.

### Example 1: Separation of extract, fractions, and active compounds from Zanthoxylum piperitum leaves

As shown in FIG. 1, the process of separating an extract, fractions, and active compounds from *Zanthoxylum piperitum* leaves is described below.

*Zanthoxylum piperitum* that was used was domestic *Zanthoxylum piperitum* collected from the southern region of Korea. The leaves of *Zanthoxylum piperitum* were washed, dried (in the shade), and then chopped. The leaves of *Zanthoxylum piperitum* were immersed in a 90% ethanol aqueous solution, followed by extraction 2 to 5 times at room temperature and then distillation under reduced pressure to obtain a *Zanthoxylum piperitum* leaf ethanol extract.

68.8 g of the *Zanthoxylum piperitum* leaf ethanol extract was suspended in 2 L of distilled water, sequentially fractionated with equal amounts of n-hexane, dichloromethane (CH₂Cl₂), ethyl acetate (EtOAc), n-butanol (n-BuOH), and water, and then concentrated under reduced pressure to obtain respective solvent fractions. Each of the solvent fractions thus obtained was analyzed for virus inhibitory activity, among which the ethyl acetate fraction showing the best antiviral activity was selected.

The ethyl acetate fraction was placed in a glass column (5x50 cm) packed with silica beads and subjected to silica gel column chromatography. As such, a mixed solution of ethyl acetate and methanol was used as an eluent, and elution was performed while changing the volume ratio of ethyl acetate/methanol from 90/10 to 60/40. Silica gel column chromatography was performed to obtain seven fractions (fraction ZPE1, 2.86 g; fraction ZPE2, 0.32 g; fraction ZPE3, 0.30 g; fraction ZPE4, 1.13 g; fraction ZPE5, 0.54 g; fraction ZPE6, 1.90 g; fraction ZPE7, 1.90 g). Virus inhibitory activity of each of the seven fractions was analyzed, among which fraction ZPE6 showing the best activity was selected.

Fraction ZPE6 was concentrated under reduced pressure, dried completely, placed in a Sephadex LH-20 column, and subjected to medium pressure liquid chromatography (MPLC). As such, elution was performed using methanol alone as the eluent. Medium pressure liquid chromatography was carried out to obtain three subfractions (subfraction ZPE6A, 24.7 mg; subfraction ZPE6B, 416 mg; subfraction ZPE6C, 65.4 mg). Virus inhibitory activity of each of the three subfractions was analyzed, among which subfraction ZPE6B showing the best activity was selected.

Subfraction ZPE6B was subjected to HPLC (eluent: methanol/water = 50:50) using a reverse phase column packed with octadecyl-functionalized silica, thereby separating active ingredient 1 and active ingredient 2.

The chemical structures of active ingredient 1 and active ingredient 2 separated above were identified through nuclear magnetic resonance spectroscopy, respectively.

### Active ingredient 1

¹H-NMR (300 MHz, DMSO-*d*₆) δ 0.9 (d, *J* = 6 Hz, CH₃), 5.2 (d, *J* = 1.5 Hz, H-1"), 6.15 (d, *J* = 2.2 Hz, H-6), 6.36 (d, *J* = 2.2 Hz, H-8), 6.95 (d, *J* = 8.2 Hz, H-5'), 7.25 (dd, *J* = 2.2 Hz and *J* = 8.2 Hz, H-6'), 7.35 (d, *J* = 2.2 Hz, H-2'); ¹³C-NMR (75 MHz, DMSO- *d*₆) δ 17.5 (C-6"), 70.0 (C-5"), 70.2 (C-3"), 70.5 (C-2"), 71.1 (C-4"), 93.6 (C-8), 98.6 (C-6), 101.8 (C-1"), 104.0 (C-10), 115.4 (C-2'), 115.6 (C-5'), 120.7 (C-6'), 121.1 (C-1'), 134.2 (C-3), 145.1 (C-3'), 148.3 (C-4'), 156.4 (C-2), 157.2 (C-9), 161.3 (C-5), 164.2 (C-7), 177.7 (C-4)

Based on the above spectroscopic analysis results, active ingredient 1 was identified as quercetin-3-O-alpha-L-rhamnoside represented by [Chemical Formula 1] below. The results of spectroscopic analysis of active ingredient 1 matched the literature values for quercetin-3-O-alpha-L-rhamnoside (quercitrin).

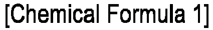

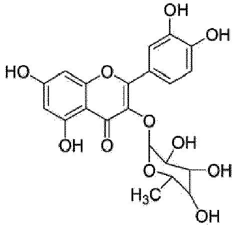

### Active ingredient 2

¹H-NMR (300 MHz, DMSO-*d*₆) δ 0.9 (d, *J* = 6 Hz, CH 3), 5.29 (1H, d, *J* = 1.5 Hz, H-1"), 6.21 (1H, d, *J* = 2.5 Hz, H-6), 6.42 (1H, d, *J* = 2.5 Hz, H-1"), 6.21 (1H, d, *J* = 2.5 Hz, H-6), 6.42 (1H, d, *J* = 2.5 Hz, H-8), 6.91 (2H, d, *J* = 8.4 Hz, H-3' and H-5'), 7.74 (2H, d, *J* = 8.4 Hz, H-2' and H-6'); ¹³C-NMR (75 MHz, DMSO-*d*₆) δ 17.4 (C-6"), 70.0 (C-5"), 70.2 (C-2"), 70.6 (C-3"), 71.0 (C-4"), 93.7 (C-8), 98.7 (C-6), 101.7 (C-1"), 104.0 (C-10), 115.3 (C-3', C-5'), 120.5 (C-1), 130.6 (C-2', C-6'), 134.2 (C-3), 156.5 (C-9), 157.2 (C-2), 159.9 (C-4'), 161.2 (C-5), 164.3 (C-4), 177.7 (C-7)

Based on the above spectroscopic analysis results, active ingredient 2 was identified as kaempferol-3-O-alpha-L-rhamnoside represented by [Chemical Formula 2] below. The results of spectroscopic analysis of active ingredient 2 matched the literature values for kaempferol-3-O-alpha-L-rhamnoside (afzelin).

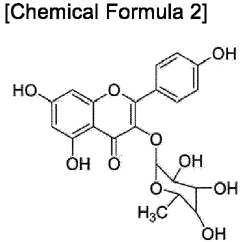

### Example 2: GC-MS analysis of ZPE6A and ZPE6C subfractions

In order to analyze the active ingredients contained in subfractions ZPE6A and ZPE6C separated in Example 1, GC-MS (gas chromatography-mass spectrometry) was performed, and the results thereof are shown in Table 1 below.

**[Table 1]**

| **Peak No.** | **Compound** | **ZPE6A** | | **ZPE6C** | |
|---|---|---|---|---|---|
| | | RT (retention time) | Area (%) | RT (retention time) | Area (%) |
| **1** | 2,4-di-*tert*-butylphenol | 14.540 | 26.69 | 14.540 | 12.09 |
| **2** | Eicosane | 15.974 | 3.99 | 14.288 | 3.67 |
| **3** | Methyl myristate | 17.977 | 4.00 | 17.978 | 4.84 |
| **4** | Methyl palmitate | 20.933 | 32.09 | 20.934 | 31.05 |
| **5** | 1,2-benzenedicarboxylic acid | 21.514 | 6.99 | 21.514 | 9.53 |
| **6** | Methyl stearate | 23.638 | 10.78 | 23.649 | 9.86 |
| **7** | 1,2-benzenedicarboxylic acid dipropyl ester | 28.674 | 15.47 | 28.674 | 17.85 |
| **8** | 4'-hydroxyacetophenone | N/D | N/D | 12.514 | 5.62 |
| **9** | Nonadecane | N/D | N/D | 15.974 | 5.49 |

Based on the results of GC-MS analysis of Table 1, the ZPE6A or ZPE6C subfraction was confirmed to contain 4'-hydroxyacetophenone represented by [Chemical Formula 3] below, 2,4-di-*tert*-butylphenol represented by [Chemical Formula 4] below, and 1,2-benzenedicarboxylic acid represented by [Chemical Formula 5] below.

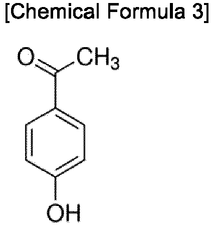

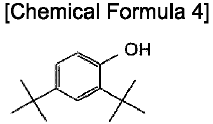

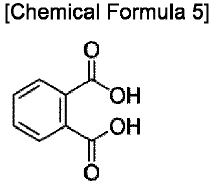

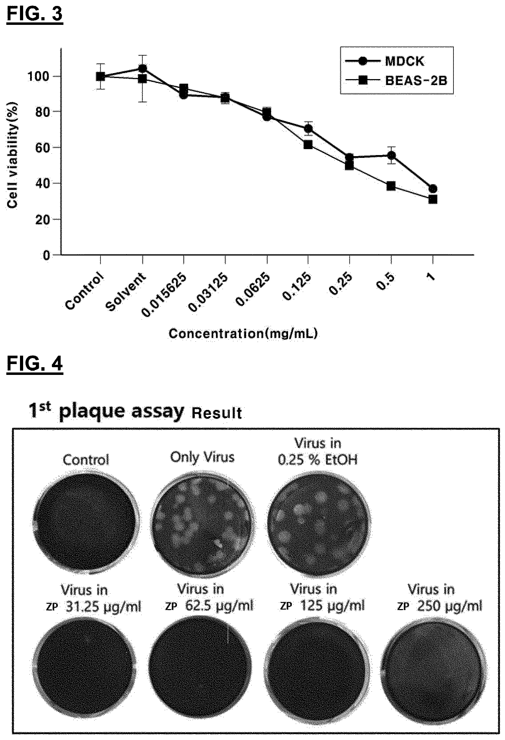

In addition, inhibitory activity of 4'-hydroxyacetophenone, 2,4-di-*tert-*butylphenol, and 1,2-benzenedicarboxylic acid separated from the ZPE6A or ZPE6C subfraction on coronavirus replication was measured. The results thereof are shown in FIG. 3.

### Example 3: Difference in amount of active compound depending on extraction conditions

In the process of obtaining the *Zanthoxylum piperitum* leaf extract according to Example 1, various extracts were prepared using different extraction solvents at different temperatures. For extracts, the amounts of active compounds (quercitrin, afzelin) were analyzed through HPLC. The analysis results thereof are shown in Table 2 below.

**[Table 2]**

| Comparison of amounts of active compounds of extracts depending on extraction conditions | | | | |
|---|---|---|---|---|
| Extraction conditions | Sample | Yield (%) | Active compound [area%, retention time: 25 min] | |
| | | | Quercitrin Afzelin | |
| Condition 1 | 90% ethanol (EtOH) extract-unheated | 5.1 | 36.47 | 12.85 |
| Condition 2 | 90% ethanol extract-heated | 6.8 | 56.44 | 4.68 |
| Condition 3 | Material obtained by removing emulsion through centrifugation of extract of Condition 2 | 5.7 | 58.02 | 4.05 |
| Condition 4 | Fraction obtained by fractionating 90% ethanol extract with dichloromethane (CH₂Cl₂) | 2.2 | 2.60 | 7.88 |
| Condition 5 | Residue excluding fraction of Condition 4 | 3.6 | 66.47 | 15.36 |
| Condition 6 | *n*-hexane extract | 1.1 | 0.41 | 4.81 |
| Condition 7 | Ethanol extract obtained by extracting extract of Condition 6 with 90% ethanol | 5.1 | 49.50 | 14.33 |
| Condition 8 | Dichloromethane (CH₂Cl₂) extract | 1.7 | 0.37 | 6.01 |
| Condition 9 | Ethanol extract obtained by extracting extract of Condition 8 with 90% ethanol | 4.5 | 59.49 | 15.91 |

As is apparent from Table 2, it can be found that the amount of the active compound is capable of varying depending on the extraction conditions.

### Example 4: Evaluation of in-vitro cytotoxicity of Zanthoxylum piperitum extract

In order to determine the optimal concentration at which the *Zanthoxylum piperitum* extract is capable of exhibiting antiviral effect without cytotoxicity, cytotoxicity was measured using MTT assay.

The cell lines that were used were Madin-Darby canine kidney (MDCK) cell line and human bronchial epithelial (BEAS-2B) cell line.

The test method was as follows.
1) 100 µl of cells were dispensed in each well of a 96-well plate at a concentration of 1×10⁴ cells/well, followed by culture at 37°C and 5% CO₂ for 24 hours.
2) After 24 hours, the cells were treated with the *Zanthoxylum piperitum* 95% ethanol extract and allowed to react at 37°C and 5% CO₂ for 4 hours.
3) The fluorescence intensity after treatment with an MTT solution was measured at 570 nm using a multi-reader (Synergy H1 Multi-mode Reader, BioTek).
4) Cell viability was calculated relative to a control group using MTT assay.

- The control group that was used was an untreated group.

The experimental results are shown in FIG. 3 and Table 3 below.

**[Table 3]**

| Cell line | IC₅₀ (µg/ml) | R² |
|---|---|---|
| MDCK (Madin-Darby Canine Kidney) | 156.1 | 0.968 |
| BEAS-2B (Human bronchial epithelial) | 145.6 | 0.997 |

As is apparent from the above results, cytotoxicity of the *Zanthoxylum piperitum* 95% EtOH extract alone was observed (there was no cytotoxicity due to a solvent). The following antiviral efficacy evaluation was performed in the concentration range without the cytotoxic effect of the *Zanthoxylum piperitum* extract (160 µg/ml > IC₅₀).

### Example 5: Confirmation of ability to inhibit intracellular virus infection - plaque reduction

Plaque reduction assay was performed in order to confirm the ability of the *Zanthoxylum piperitum* extract to inhibit intracellular virus infection.

Plaque is a single-layered cell region that has undergone cellular degeneration due to virus infection, and is applied to virus quantification under the theory that one infectious virus particle forms one plaque. In this test, antiviral efficacy of the *Zanthoxylum piperitum* extract was evaluated by measuring the extent of intracellular plaque formation of the *Zanthoxylum piperitum* extract.

The cell line that was used was the Madin-Darby canine kidney (MDCK) cell line.

The test method was as follows.

The experiment was prepared according to the following protocol.
- Cells: MDCK (Madin-Darby Canine Kidney cells)
- Virus: Influenza (A/California/07/2009/H1N1)
- Virus concentration:
   The first trial was 3×10² PFU/mL

The second trial was 1×10³ PFU/mL
- Sample: only virus/solvent/virus+*Zanthoxylum piperitum* extract (N=4)

*Zanthoxylum piperitum* extract: Preparation of a stock at a concentration of 100 mg/ml in ethanol (≥99.45%)

Solvent: 0.25% EtOH based on 250 µg/ml of *Zanthoxylum piperitum*

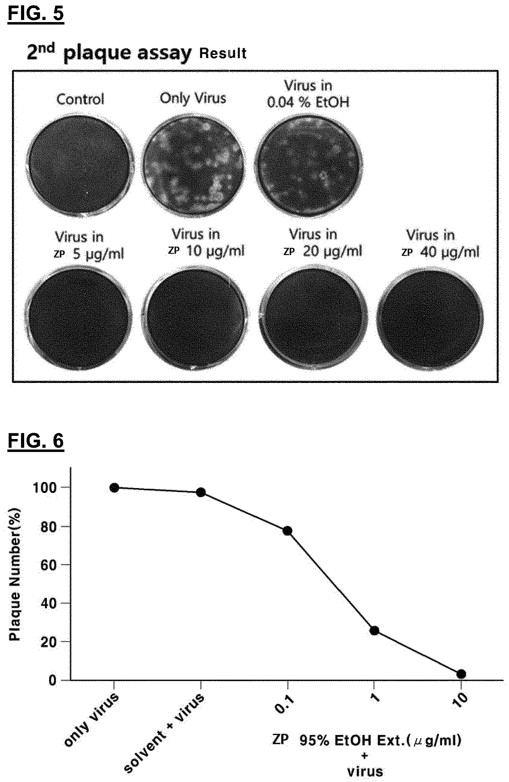

Virus infection conditions: 100 µl, 1.5 hours, 37°C, 5% CO₂
(1) The results of primary plaque assay are shown in FIG. 4 and the results of secondary plaque assay are shown in FIG. 5. The experimental results demonstrated that there was almost no antiviral effect due to the solvent (0.04% EtOH). It was confirmed that no plaque was formed at a concentration ranging from 5 µg/mL to 31.25 µg/mL at which there was almost no cytotoxic effect of the *Zanthoxylum piperitum* extract (Cell viability > 85% at 31.25 µg/mL of *Zanthoxylum piperitum* 95% EtOH extract).
(2) Therefore, the following additional experiment was performed because it was necessary to confirm results at concentrations of 5 µg/mL or less.

- Cells: MDCK (Madin-Darby Canine Kidney cells)
- Virus: Influenza (A/California/07/2009/H1N1)
- Virus concentration: 1×10³ PFU/mL
- Sample: only virus/solvent/virus+*Zanthoxylum piperitum* extract (N=4)

*Zanthoxylum piperitum* extract: Preparation of a stock at a concentration of 20 mg/ml in DMSO (≥99%)

Solvent: 0.05% DMSO based on 10 µg/ml *of Zanthoxylum piperitum*
- Virus infection conditions: 100 µl, 1.5 hours, 37°C, 5% CO₂

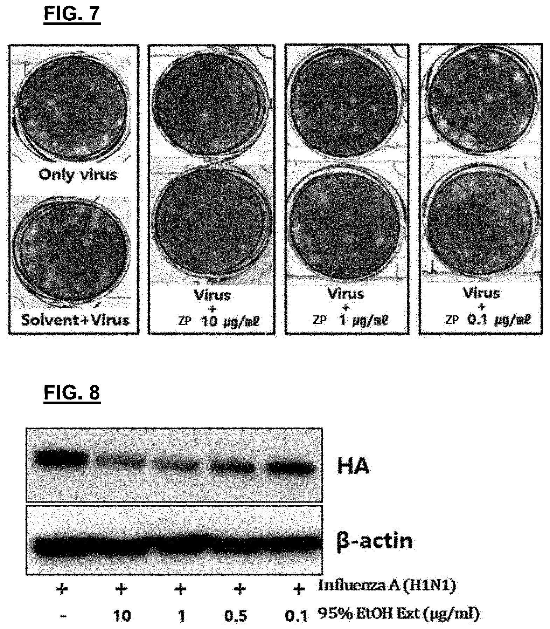

The experimental results are shown in FIGS. 6 and 7. It was confirmed that there was almost no antiviral effect due to the solvent and also that the *Zanthoxylum piperitum* extract alone had an antiviral effect. In the group treated with the *Zanthoxylum piperitum* 95% EtOH extract, plaque was not formed in a concentration-dependent manner, indicating inhibitory efficiency of about 95% or more at a concentration of 10 µg/ml.

### Example 6: Confirmation of ability to inhibit intracellular virus infection - Western blot

A difference in the expression level of viral protein in cells depending on the presence or absence of the *Zanthoxylum piperitum* extract was measured using Western blot assay.

The *Zanthoxylum piperitum* extract was used along with virus to treat the cells, the expression level of viral protein in the infected cells depending on the concentration thereof was measured through Western blot, and the virus inactivation effect and virus infection inhibitory effect of the *Zanthoxylum piperitum* extract were evaluated.

The cell line that was used was the Madin-Darby canine kidney (MDCK) cell line.

The test method was as follows.
1) A 6-well cell culture dish in which MDCK cells were dispensed at 1×10⁸ cells/well was treated with a mixture of 2×10⁵ PFU/ml of virus and *Zanthoxylum piperitum* extract for 1.5 hours. The virus that was used was influenza (A/California/07/2009/H1N1).
   - Control groups that were used were a group treated with only virus and an untreated group.
2) After 1.5 hours, MDCK cells were washed with PBS and cultured for 2 days in DMEM containing 0.3% BSA (bovine serum albumin) to express viral proteins.
   After 2 days, the cells were treated with a cell lysis buffer (RIPA buffer), after which the cells were collected by scraping each well of the plate in which the cells were cultured using a scraper, followed by centrifugation to separate the supernatant.
3) Quantitative protein analysis of the supernatant was performed using a BCA protein analysis kit, and proteins were separated using SDS-PAGE and transferred to a PVDF membrane.
4) The PVDF membrane to which the protein had been transferred was blocked with a 5% skim milk solution for 1 hour, treated with a primary antibody specifically recognizing the virus surface protein (e.g. hemagglutinin in the case of influenza), and then allowed to react overnight at 4°C.
5) After reaction, the cells were washed thoroughly with a TBST (containing 0.1% Tween 20) buffer, treated with a secondary antibody (HRP conjugated), and allowed to react at room temperature for 2 hours.
6) The cells were washed thoroughly with a TBST (containing 0.1% Tween 20) buffer, and treated with an enhanced chemiluminescence (ECL) solution to determine the band expressed in Chemi-doc imager.
   - In order to compare the expression level of viral protein in the same number of cells, Western blot for β-actin, which is a protein expressed at a similar level in all cells, was performed simultaneously in the same manner.

The experimental results are shown in FIG. 8. It was confirmed that the group treated with both the virus and the *Zanthoxylum piperitum* 95% EtOH extract inhibited the expression of viral protein in a concentration-dependent manner compared to the group treated with only virus. It was also confirmed that there was a positive linear correlation with the results of plaque reduction assay.

### Example 7: Evaluation of anti-inflammatory IκBα degradation inhibitory efficacy

In order to evaluate the anti-inflammatory effect of the *Zanthoxylum piperitum* extract (95% EtOH extract), the extent of inhibition of IκBα degradation depending on the concentration of the *Zanthoxylum piperitum* 95% EtOH extract was measured. IκBα is degraded within cells when inflammatory response is activated, and activates NF-kB transcription to thus increase the secretion of proinflammatory cytokines.

The experimental method was as follows.
- Cells: RAW264.7 (mouse macrophage cells)
- Sample: Control/LPS/LPS+*Zanthoxylum piperitum* extract

*Zanthoxylum piperitum* extract: Preparation of a stock at a concentration of 20 mg/ml in DMSO (≥99%)
LPS (lipopolysaccharide): Lipopolysaccharide, which is a component that causes bacterial inflammation
1 hour after pretreatment with *Zanthoxylum piperitum,* the cells were treated with LPS for 6 hours (in SF media)

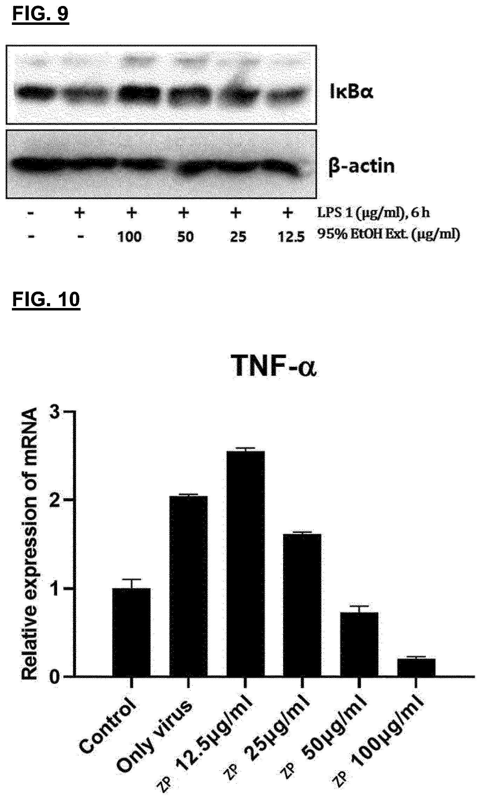

The experimental results are shown in FIG. 9. Treatment with only LPS induced IκBα degradation and thus faint bands were observed. It was confirmed that IκBα degradation by LPS was inhibited in the group pretreated with the *Zanthoxylum piperitum* 95% EtOH extract (in which there was a significant difference in protein bands in a concentration-dependent manner).

### Example 8. Evaluation of cytokine expression level of Zanthoxylum piperitum extract through RT-PCR

In order to confirm the anti-inflammatory effect of the *Zanthoxylum piperitum* extract (95% EtOH extract), the intracellular cytokine mRNA expression level depending on the concentration of the *Zanthoxylum piperitum* 95% EtOH extract was measured. As TNF-α, IL-6, and IL-1β are proinflammatory cytokines and play a major role in acute inflammatory response, the expression levels thereof were measured.

The experimental method was as follows.
- Cells: RAW264.7 (mouse macrophage cells)
- Virus: Influenza (A/California/07/2009/H1N1)
- Sample: Control/virus/virus+*Zanthoxylum piperitum* extract

*Zanthoxylum piperitum* extract: Preparation of a stock at a concentration of 20 mg/ml in DMSO (≥99%)
1 hour after virus pretreatment, the cells were treated with the *Zanthoxylum piperitum* extract for 16 hours.

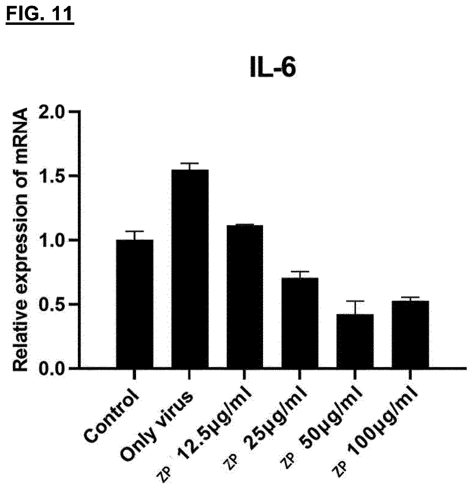

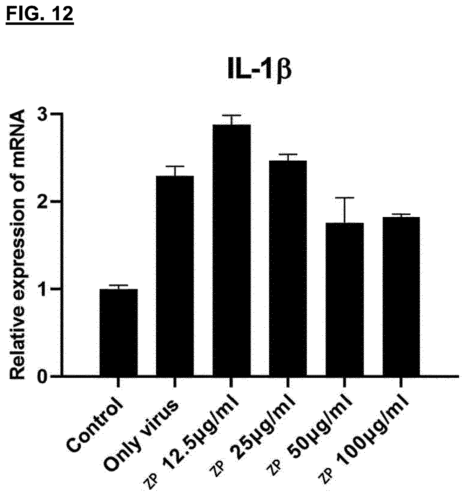

The experimental results are shown in FIGS. 10 to 12.

In the case of treatment with only virus, the expression level of TNF-α/IL-6/IL-1β was increased a maximum of 2 times compared to the control group. Upon treatment with the *Zanthoxylum piperitum* 95% EtOH extract, the expression level of TNF-α/IL-6/IL-1β was inhibited in a concentration-dependent manner.

Based on the experimental results of Examples 4 to 6, the antiviral efficacy of the *Zanthoxylum piperitum* extract on inhibiting intracellular influenza infection and replication was confirmed. In addition, based on the results of Examples 7 and 8, the efficacy of the *Zanthoxylum piperitum* extract on inhibiting IκBα degradation and cytokine (TNF-α, IL-6, IL-10) secretion was confirmed, indicating effective alleviation of excessive inflammatory response (cytokine storm) caused by virus infection.

### [Formulation Examples]

In the following Formulation Examples, preparation of a pharmaceutical composition or a food composition containing the extract, fraction, or active compound (quercitrin, afzelin, or a mixture thereof) of the present invention as an active ingredient is described. The following Formulation Examples are set forth to illustrate the present invention and are not to be construed as limiting the present invention.

### Formulation Example 1: Preparation of pharmaceutical composition

### Formulation Example 1-1: Preparation of tablets

10 g of povidone was dissolved in 50 g of ethanol to prepare a binding solution, after which 150 g of an active ingredient was added to the binding solution and homogeneously stirred. 130 g of silicified microcrystalline cellulose, 15 g of croscarmellose sodium, and 20 g of colloidal silicon dioxide were mixed together, and the homogeneously stirred binding solution containing the active ingredient was added thereto, followed by a granulation process to obtain granules, which were then dried and sized. The resulting granules were added with 15 g of crospovidone and mixed therewith, and 3.5 g of colloidal silicon dioxide and 6.5 g of magnesium stearate were added thereto, followed by a lubrication process to make tablets.

### Formulation Example 1-2: Preparation of tablets

19 g of povidone was dissolved in 50 g of ethanol to prepare a binding solution, after which 150 g of an active ingredient was added to the binding solution and homogeneously stirred. 143 g of silicified microcrystalline cellulose, 15 g of croscarmellose sodium, and 20 g of Neusilin were mixed together, and the homogeneously stirred binding solution containing the active ingredient was added thereto, followed by a granulation process to obtain granules, which were then dried and sized. The resulting granules were added with 15 g of crospovidone and mixed therewith, and 5 g of colloidal silicon dioxide and 8 g of magnesium stearate were added thereto, followed by a lubrication process to make tablets.

### Formulation Example 1-3: Preparation of tablets

12.5 g of povidone was dissolved in 50 g of ethanol to prepare a binding solution, after which 150 g of an active ingredient was added to the binding solution and homogeneously stirred. 62.5 g of silicified microcrystalline cellulose, 65 g of lactose hydrate, 15 g of croscarmellose sodium, and 20 g of colloidal silicon dioxide were mixed together, and the homogeneously stirred binding solution containing the active ingredient was added thereto, followed by a granulation process to obtain granules, which were then dried and sized. The resulting granules were added with 15 g of crospovidone and mixed therewith, and 3.5 g of colloidal silicon dioxide and 6.5 g of magnesium stearate were added thereto, followed by a lubrication process to make tablets.

### Formulation Example 1-4: Preparation of tablets

19 g of povidone was dissolved in 33.5 g of ethanol to prepare a binding solution, after which 150 g of an active ingredient was added to the binding solution and homogeneously stirred. 105 g of silicified microcrystalline cellulose (in which the amount of silicified microcrystalline cellulose that was added equaled the amount of ethanol contained in the main ingredient), 17.5 g of croscarmellose sodium, and 20 g of Neusilin were mixed together, and the homogeneously stirred binding solution containing the active ingredient was added thereto, followed by a granulation process to obtain granules, which were then dried and sized. The resulting granules were added with 17.5 g of crospovidone and 33 g of silicified microcrystalline cellulose and mixed therewith, and 7.5 g of colloidal silicon dioxide and 8 g of magnesium stearate were added thereto, followed by a lubrication process to make tablets.

### Formulation Example 1-5: Preparation of coated tablets

Coated tablets were prepared from 755 mg of the tablets prepared in Formulation Examples 1-1 to 1-4 (containing 300 mg of the active ingredient) using a typical film-coating system (Opadry^{®}, Opadry200^{®}, or Opaglos^{®}).

### Formulation Example 1-6: Preparation of injectable solution

1 g of an active ingredient, 0.6 g of sodium chloride, and 0.1 g of ascorbic acid were dissolved in distilled water to make 100 ml. This solution was placed in a bottle and sterilized by heating at 20°C for 30 minutes.

### Formulation Example 2: Preparation of health food

### Formulation Example 2-1: Preparation of beverage

1000 mg of an active ingredient, 1000 mg of citric acid, 100 g of oligosaccharide, and 1 g of taurine were mixed together, and purified water was added thereto so that the total volume was 900 ml. After stirring and heating at 85°C for about 1 hour, the resulting solution was filtered, placed in a sterile 2 L container, sealed, and sterilized to make a beverage.

## Claims

1. A pharmaceutical composition for preventing, alleviating, or treating coronavirus infection comprising a *Zanthoxylum piperitum* leaf extract, a fraction thereof, or a compound separated therefrom as an active ingredient.

2. The pharmaceutical composition of claim 1, wherein the extract is extracted with a 60% to 99% ethanol aqueous solution.

3. The pharmaceutical composition of claim 1, wherein the extract is extracted with a 90% to 99% ethanol aqueous solution.

4. The pharmaceutical composition of claim 1, wherein the fraction is an ethyl acetate fraction obtained by suspending the *Zanthoxylum piperitum* leaf extract in distilled water and then performing sequential fractionation with n-hexane, dichloromethane, ethyl acetate, and n-butanol.

5. The pharmaceutical composition of any one of claims 1 to 4, wherein the *Zanthoxylum piperitum* leaf extract or the ethyl acetate fraction comprises, as an active ingredient, at least one compound selected from the group consisting of quercetin-3-O-alpha-L-rhamnoside represented by [Chemical Formula 1] below, kaempferol-3-O-alpha-L-rhamnoside represented by [Chemical Formula 2] below, 4'-hydroxyacetophenone represented by [Chemical Formula 3] below, 2,4-di-tert-butylphenol represented by [Chemical Formula 4] below, and 1,2-benzenedicarboxylic acid represented by [Chemical Formula 5] below:

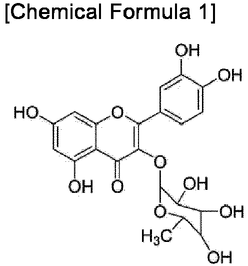

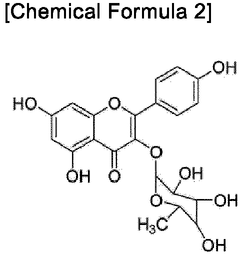

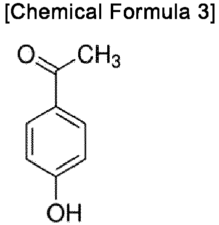

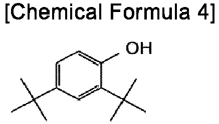

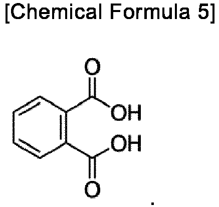

6. The pharmaceutical composition of claim 1, wherein the coronavirus is at least one selected from the group consisting of porcine transmissible gastroenteritis virus (TGEV), porcine epidemic diarrhea virus (PEDV), canine coronavirus, bovine coronavirus, SARS coronavirus (SARS-CoV), and Corona-19 virus (SARS-CoV-2).

7. The pharmaceutical composition of claim 1, wherein the pharmaceutical composition comprises a pharmaceutically acceptable carrier, excipient, or diluent.

8. The pharmaceutical composition of claim 1, wherein the pharmaceutical composition is administered orally in any form selected from the group consisting of a powder, a granule, a tablet, a capsule, and a liquid form.

9. A food composition for preventing or ameliorating coronavirus infection comprising a *Zanthoxylum piperitum* leaf extract, a fraction thereof, or a compound separated therefrom as an active ingredient.
